Europäisches Patentamt

European Patent Office

Office européen des brevets

(19)

(11) Veröffentlichungsnummer: **0 016 315**
**B1**

(12) **EUROPÄISCHE PATENTSCHRIFT**

(45) Veröffentlichungstag der Patentschrift: **09.03.83**

(21) Anmeldenummer: **80100396.3**

(22) Anmeldetag: **25.01.80**

(51) Int. Cl.³: **A 61 C 5/10,** A 61 K 6/00,
B 23 K 35/24, C 04 B 37/00

(54) **Mittel und Verfahren zum Verbinden von Dentalmetallteilen mit Dentalporzellan bzw. mit anderen Dentalmetallteilen.**

(30) Priorität: **08.03.79 US 18767**
**04.04.79 US 26785**

(43) Veröffentlichungstag der Anmeldung:
**01.10.80 Patentblatt 80/20**

(45) Bekanntmachung des Hinweises auf die Patenterteilung:
**09.03.83 Patentblatt 83/10**

(84) Benannte Vertragsstaaten:
**AT BE CH DE FR GB IT NL SE**

(56) Entgegenhaltungen:
**CH - A - 437 819**
**DE - A - 2 040 139**
**DE - A - 2 531 113**
**DE - A - 2 729 488**
**DE - C - 622 465**
**DE - C - 873 375**
**DE - C - 888 668**
**US - A - 3 585 064**
**US - A - 4 162 163**

(73) Patentinhaber: **Etablissement Dentaire IVOCLAR**
**FL-9494 Schaan (LI)**

(72) Erfinder: **Shoher, Itzhak, Dr.**
**50, Shlomo Hamelech St.**
**Tel Aviv 64386 (IL)**

(74) Vertreter: **Splanemann, Rainer et al,**
**Patentanwälte R. Splanemann Dr. B. Reitzner Tal 13**
**D-8000 München 2 (DE)**

Courier Press, Leamington Spa, England.

# Mittel und Verfahren zum Verbinden von Dentalmetallteilen mit Dentalporzellan bzw. mit anderen Dentalmetallteilen

Die Erfindung betrifft ein Mittel, die Verwendung eines Mittels und ein Verfahren zum Verbinden von Dentalmetallteilen, insbesondere aus Edelmetallen oder Edelmetall-Legierungen, mit Dentalporzellan bzw. mit anderen Dentalmetallteilen.

Bei der Wiederherstellung von Zähnen, insbesondere bei der Anfertigung von Kronen und Brücken, wird der Metallunterbau mit Dentalporzellan schichtweise bedeckt, worauf Schicht für Schicht bei relativ hohen Temperaturen aufgebrannt wird. Gewöhnlich besteht der Metallunterbau aus Edelmetallen mit einem hohen Goldanteil. Das Dentalporzellan setzt sich im Allgemeinen aus Feldspat, Quarz und Kaolin zusammen und enthält noch geringe Anteile anderer Substanzen. Ein klinisch zufriedenstellender Verbund zwischen Metallunterbau und Porzellan ist u.a. abhängig von den thermischen Ausdehnungskoeffizienten der verwendeten Materialien. Da das Porzellan schichtweise auf den verwendeten Materialien. Da das Porzellan schichtweise auf den Metallunterbau bei erhöhten Temperaturen aufgebrannt und bei Raumtemperatur abgekühlt wird, entstehen Spannungen zwischen den einzelnen Materialschichten und zwischen Metall und Porzellan. Ein befriedigender Verbund zwischen der Metallunterlage und dem Porzellan wird generell mechanischen Retentionen oder den Van der Waals'schen Kräften zugeschrieben, während der chemische Verbund gering ist. Die Kohäsionskräfte, die aus einem solchen Verbund herrühren, sind ungleich schwächer als echte chemische Bindungen.

In der Vergangenheit wurden verschiedentlich schon Versuche unternommen, ein Bindemittel als Zwischenschicht auf ein Metallgerüst vor dem Brennen aufzubringen, um den Grad der Adhäsion zwischen Zahnporzellan und Metall zu erhöhen. Als Beispiel wird die DE—A—25 31 113 genannt, in der die Bildung eines Oxids wie z.B. von Zinnoxid auf der Oberfläche des Metalls vor dem Brennen vorgeschlagen wird. Obwohl die Oxidschicht chemisch an das Metallgerüst gebunden ist, wird der chemische Verbund zwischen Dentalporzellan und Metall nicht wesentlich verbessert, und unter klinischen Bedingungen ist die Verbindung nicht unzerbrechlich. "Klinisch unzerbrechlich" ist eine Verbindung, die im Mund unter Kaubelastung auch nach längerer Zeit keine Risse oder abgebrochene Stellen aufweist.

Es ist ferner bekannt, daß die Adhäsion durch eine mechanische Vorbehandlung des Metallgerüsts verbessert werden kann. Die Erhöhung der Adhäsion ist zurückzuführen auf die Erhöhung der Van der Waals'schen Kräfte. Ferner ist aus der US—A—35 85 064 die Herstellung von Verbindungen von schmelzbarem Porzellan mit einer Edelmetallunterlage bekannt, wobei als Bindemittel ein Gemisch aus Goldpulver, Silberpulver und einem Flußmittel verwendet wird, das mit Äthylenglykol vermischt ist. Als Flußmittel sind speziell Natriumfluorid und Borax genannt. Hinweise auf die Verwendung von Edelmetallhalogeniden als Bindemittel finden sich nicht. Nach diesem Verfahren muß die Bindemittelschicht zweimal eingebrannt werden.

Die DE—C—622 465 beschreibt die Herstellung einer Jacket-Krone. Üblicherweise wird ein Gipsmodell des zu überkronenden Zahnes mit einer dünnen Platinfolie ummantelt. Auf diese Platinfolie wird Zahnporzellan aufgetragen, welches zur fertigen Krone gebrannt wird. Die Platinfolie wird anschliessend wieder entfernt; sie dient lediglich dazu, den Platz für den Befestigungszement bereitzustellen, mit dem die Jacket-Krone auf dem Zahnstumpf befestigt wird. Anstelle dieser Platinfolie wird bei dem Verfahren nach der DE—C—622 465 eine Platinammoniumchlorid-Lösung auf das Modell aus einer Porzellanmasse aufgestrichen, die in einen dünnen festhaftenden Platinüberzug umgewandelt wird, auf welchem das Porzellanmehl zum Brennen der Krone aufgetragen wird.

Der Erfindung liegt die Aufgabe zugrunde, eine klinisch unzerbrechliche Verbindung zwischen Metallunterbau und Dentalporzellan bzw. zwischen Dentalmetallteilen untereinander zu schaffen und die Spannungen zwischen den einzelnen Schichten zu verringern.

Gegenstand der Erfindung ist somit die Verwendung eines Mittels, enthaltend 1 bis 100 Gew.-%, eines oder mehrerer Edelmetallhalogenide und 0 bis 99 Gew.-% einer feinteiligen Edelmetallkomponente, zur Verbindung von Dentalmetallteilen, insbesondere aus Edelmetallen oder Edelmetall-Legierungen, mit Dentalporzellan bzw. anderen Dentalmetallteilen bei Zahnrestaurationen.

Gegenstand der Erfindung ist ferner ein Verfahren zum Verbinden von Dentalmetallteilen mit (a) Dentalporzellan bzw. mit (b) anderen Dentalmetallteilen bei Zahnrestaurationen, wobei ein Bindemittel auf eine Metallunterlage aufgebracht und gesintert bzw. gebrannt wird und anschließend im Fall (a) Dentalporzellan aufgetragen und aufgebrannt wird; dieses Verfahren ist durch die Verwendung des vorstehend angegebenen Bindemittels gekennzeichnet.

Durch die Erfindung wird ein klinisch unzerbrechlicher Verbund zwischen Metall und Dentalporzellan bzw. zwischen zwei Dentalmetallteilen geschaffen, wobei es theoretisch noch nicht vollständig klar ist, warum dies geschieht. Man erklärt sich die Ausbildung des Verbundes so, daß zunächst ein chemischer Verbund zwischen der Metallunterlage und dem Bindemittel geschaffen wird. Das Bindemittel reagiert dann mit dem anderen Dentalmetallteil bzw. mit dem Dentalporzellan. Im letzteren Fall werden wahrscheinlich die Spannungen im Dentalporzellan abgebaut, und es wird eine chemische Verbindung zum Dentalporzellan selbst hergestellt.

Gegenstand der Erfindung ist ferner ein Mittel zum Verbinden von Dentalmetallteilen mit Dentalporzellan bzw. anderen Dentalmetallteilen bei Zahnrestaurationen, das dadurch gekennzeichnet ist, daß es 1 bis 99,9 Gew.-% eines oder mehrerer Edelmetallhalogenide und 0,1 bis 99 Gew.-% einer feinteiligen Edelmetallkomponente enthält.

Die verwendeten Edelmetallhalogenide sind vorzugsweise die Chloride und/oder Fluoride, obwohl auch die Bromide und die Jodide verwendet werden können. Vorzugsweise werden als Edelmetallhalogenide Silberchlorid, Goldchlorid und/oder Goldfluorid verwendet.

Die Edelmetallkomponente ist überwiegend aus Edelmetallen oder deren Legierungen zusammengesetzt, wobei vorzugsweise Gold und/oder Silber verwendet werden. Ferner sind Palladium und/oder Platin geeignet, während Rhodium, Osmium und Iridium weniger bevorzugt werden. Die feinteilige Edelmetallkomponente kann in jeder brauchbaren Form verwendet werden, z.B. in Form von Plättchen, Granulaten oder Pulvern. Vorzugsweise liegt die Teilchengröße der Edelmetallkomponente zwischen 1 und 60 $\mu$m, wobei Teilchen unter 10 $\mu$m bevorzugt werden. Ein optimaler Teilchengrößenbereich liegt zwischen 1 und 10 $\mu$m. Dabei können die Edelmetallteilchen entweder bereits in feinteiliger Form bezogen oder aber auch mit geeigneten Zerkleinerungsgeräten auf die gewünschte Größe gebracht werden.

Die Edelmetallkomponente enthält vorzugsweise wenigstens 50 Gew.-% Gold. während der Rest zu 0 bis 45 Gew.-% aus einem oder mehreren der Metalle Silber, Platin, Palladium oder Rhodium und zu 0 bis 5 Gew.-% aus einem oder mehreren der Nichtedelmetalle Kupfer, Zink, Eisen, Zinn, Cadmium, Mangan, Germanium, Magnesium, Kobalt oder Nickel zusammengesetzt ist.

Der erfindungswesentliche Bestandteil des Bindemittels ist jedoch das Edelmetallhalogenid. Elementare Edelmetallteilchen, wie z.B. Gold oder Silber, ergeben nicht die guten Resultate. Es ist zur Zeit noch nicht klar, warum Edelmetallhalogenide allein oder in Kombination mit Edelmetallen mit dem Dentalporzellan bzw. mit den Dentalmetallteilen reagieren und nach dem Brennen einen klinisch unzerbrechlichen Verbund ergeben, der chemischer Natur ist, während dies bei den Edelmetallteilchen allein nicht der Fall ist. Die Edelmetallhalogenide sind als Pulver oder in kristalliner Form erhältlich, wobei ihre Teilchengröße innerhalb oder unterhalb des gewünschten Teilchengrößenbereichs der Metallkomponente liegt. Zum Beispiel ist Goldchlorid pulverförmigkristallin als HAuCl$_4$ erhältlich und kann unmittelbar in dieser Form verwendet werden. Obwohl das Edelmetallhalogenid auch in Mengen von 100% verwendet werden kann, soll vorzugsweise aber ein Edelmetall mit verwendet werden, insbesondere Gold oder eine Legierung mit einem hohen Goldgehalt, damit der Hintergrund des Porzellanüberzugs den gewünschten goldfarbenen Ton erhält.

Das Bindemittel kann allein oder zusammen mit Dispersions-und/oder Trägerstoffen verwendet werden. Vorzugsweise wird das erfindungsgemäß Bindemittel zusammen mit einem Trägerstoff verwendet, so daß es kontrollierbar auf die Metallunterlage aufgebracht werden kann, beispielsweise durch Spritzen, Pinseln, Tauchen oder Sprühen. Der Trägerstoff ist vorzugsweise durch Hitzeeinwirkung entfernbar, d.h. er soll während des Brennens vorzugsweise rückstandsfrei verbrennen. Entsprechendes gilt für das Dispersionsmittel, wofür beispielsweise die bekannten Wasserdetergentien verwendet werden können. Als Trägerstoffe werden vorzugsweise organische Stoffe mit Klebeigenschaften verwendet, die auch mit geeigneten Lösungsmitteln verdünnt werden können. Wird kein Dispersions- oder Trägerstoff verwendet, so kann das Bindemittel in geeigneter Weise auf die Metalloberfläche gebracht werden, beispielsweise durch Aufbringen des Pulvers oder einer wäßrigen bzw. organischen Dispersion.

Für die Metallunterlage können die üblichen Dentalmetalle bzw. Dentallegierungen verwendet werden. Im allgemeinen entspricht die Zusammensetzung der Metallunterlage der Zusammensetzung der feinteiligen Edelmetallkomponente, d.h. die Metallunterlage ist vorzugsweise aus einem oder mehreren der Edelmetalle Gold, Silber, Platin oder Palladium zusammengesetzt.

Nachdem man das erfindungsgemäße Bindemittel, gegebenenfalls zusammen mit einem Dispersionsmittel und/oder einem Trägerstoff auf das Dentalmetallteil aufgebracht hat, wird es im allgemeinen bei Temperaturen im Bereich von 870 bis 1080°C gesintert bzw. gebrannt, wobei natürlich auch Temperaturen unterhalb oder oberhalb dieses Bereiches angewendet werden können, was unter anderem von der Zusammensetzung des Bindemittels bzw. der Unterlage abhängt. Ein optimaler Temperaturbereich liegt zwischen 1010 und 1040°C. Das Bindemittel wird gewöhnlich 1 bis 25 min. gesintert. Die Sintertemperatur hängt auch von der Art des verwendeten Edelmetallhalogenids ab, d.h. dieses kann eine Edelmetallunterlage auch schon bei Temperaturen unter 870°C benetzen. Bei der Herstellung einer Krone, einer Brücke oder eines künstlichten Zahnes, wird es häufig vorgezogen, die Brenntemperatur so zu wählen, daß das Bindemittel nur einen Film von unregelmäßig geformten Teilchen auf der Metallunterlage bildet. Ein für diese Zwecke optimaler Temperaturbereich liegt zwischen 980 und 1024°C. Auf die so vorbehandelte Oberfläche wird dann das Dentalporzellan aufgebrannt. Die Brenntemperatur für Dentalporzellan hängt im allgemeinen von dessen Zusammensetzung ab und kann im Bereich von 870 bis 1000°C liegen.

Als Dentalmetallteile, auf die das erfindungsgemäße Bindemittel aufgebrannt wird, kann man beispielsweise Edelmetallfolien, vorzugsweise Platinfolien, verwenden. Es können mehrere gleiche oder verschiedene Schichten Bindemittel auf ein Dentalmetallteil aufgesintert werden.

Das Dentalporzellan ergibt nach der Durchführung des erfindungsgemäßen Verfahrens mit der Edelmetallunterlage eine unzerbrechliche Verbindung. Man nimmt an, daß das aufgebrannte Bindemittel mit dem Dentalporzellan chemisch reagiert und so die unzerbrechliche Verbindung herstellt. Es ist noch nicht ganz klar, ob die Edelmetallchloride oder -fluoride sich zersetzen oder zich beim Brennen verflüchtigen. Es ist jedoch erwiesen, daß die Edelmetallhalogenide für die unzerbrechliche Verbindung verantwortlich sind. Als unzerbrechlich wird eine Verbindung definiert, die unter klinischen Bedingungen die Trennung der einzelnen Teile voneinander nicht ermöglicht. Man kann die Festigkeit der Verbindung leicht bestimmen, indem man eine Kraft senkrecht auf die Oberfläche des Verbundgegenstandes einwirken läßt, bis dieser bricht. Man kann dann visuell feststellen, ob eine Trennung der Schichten stattgefunden hat.

Es werden hauptsächlich porzellanbeschichtete, gegossenen Metallkronen zur Zahnrestauration verwendet. Der relativ dicke Metallunterbau wird hergestellt, indem man vom präparierten Zahn einen Abdruck aus Wachs herstellt, diesen in Einbettmasse einbettet, das Wachs ausbrüht und mit Metall ausgießt. Danach wird auf den Metallunterbau das Dentalporzellan schichtweise aufgetragen und gebrannt. Das verwendete Metall ist vorzugsweise ein Edelmetall bzw. eine Edelmetalllegierung, worin der Goldanteil überwiegt.

Die Dicke des gegossenen Metallunterbaus liegt in der Größenordnung von 0,2 bis 0,5 mm und ist durch die verwendeten Edelmetalle relativ teuer. Außerdem hat ein zu starker Metallunterbau zur Folge, daß für die Dentalporzellanbeschichtung wenig Platz bleibt.

Eine Jacketkrone ist deshalb vorzuziehen. Sie ist allen anderen Restaurationen ästhetisch überlegen und vom natürlichen Zahn nicht zu unterscheiden. Meistens ist sie jedoch nur für den Frontzahnbereich anwendbar, wo die Ästhetik im Vordergrund steht. Der eingeschränkte Gebrauch ist zurückzuführen auf die mangelnde Festigkeit des verwendeten Dentalporzellans. Eine Jacketkrone wird im allgemeinen hergestellt, indem man eine Platinfolie dem präparierten Zahnstumpf anpaßt, die Folie abzieht, mit Porzellan beschichtet und brennt. Die Platinfolie wird dann wieder entfernt und die Jacketkrone einzementiert. Nach der DE—A—25 31 113 braucht die Platinfolie nicht entfernt zu werden, sondern bleibt zur Verstärkung des Dentalporzellans erhalten. Durch die Metalloxidschicht bzw. die Zinnoxidschicht soll das Dentalporzellan mit der Platinfolie verbunden werden.

Das Auftragen des Bindemittels auf eine Edelmetallunterlage und die weiteren Verfahrensschritte schließen selbstverständlich auch ein, daß man unter Edelmetallunterlage auch Edelmetallfolien, insbesondere eine Platinfolie, zu verstehen hat. Die Formdicke und die Zusammensetzung der Edelmetallunterlage hängen davon ab, was hergestellt werden soll, zum Beispiel Kronen, Jacketkronen oder andere Zahnrestaurationen.

Durch Auftragen des Bindemittels auf z.B. eine Platinfolie, Brennen, des Bindemittels und anschließendem schichtweisen Auftragen und Brennen von Dentalporzellan wird so mit Hilfe des erfindungsgemäßen Bindemittels und Verfahrens eine verstärkte Jacketkrone geschaffen, die derjenigen gemäß DE—A—25 31 113 bezüglich Festigkeit weit überlegen ist.

Auf die Edelmetallunterlage kann man eine Schicht des Bindemittels auftragen und brennen, es können aber auch mehrere Schichten aufgetragen und gebrannt werden. Verwendet man als Edelmetallunterlage eine Edelmetallfolie, insbesondere eine Platinfolie, so wird mindestens eine Schicht aufgebrannt, vorzugsweise aber zwei Schichten, auch eine Vielzahl von Schichten ist möglich. Die Gesamtdicke der Schichten wird bestimmt von der Handhabbarkeit der Platinfolie. Eine zu dicke Schicht macht die Folie zu steif, so daß sie dem Zahnstumpf schlecht angepaßt werden kann.

Man kann auf eine Platinfolie das Bindemittel auftragen und brennen, schon bevor die Folie dem präparierten Stumpf angepaßt wird. Die Platinfolie kann also schon mit einer oder mehreren Schichten des eingebrannten Bindemittels in den Handel kommen. Dies gilt ebenso für alle anderen denkbaren Edelmetallunterlagen.

Verwendet man mehrere Schichten, so brauchen diese nicht gleich zu sein. Eine Schicht kann aus einem Bindemittel aus 1 bis 100% Edelmetallhalogeniden mit 0 bis 99% Edelmetallen bestehen, die anderen Schichten können gleich oder verschieden von der ersten Schicht sein.

Die Erfindung ist anhand der nachstehenden Beispiele erläutert. Alle Prozentangaben beziehen sich auf das Gewicht.

Beispiel 1
Auf einen Metallunterbau (dünnes Edelmetall, 100% Pt) wird ein Bindemittel folgender Zusammensetzung aufgebracht:

A) Edelmetallhalogenid: 1,4% pulverförmiges AgCl
B) Edelmetallkomponente: Mischung

feiner Teilchen (zwischen 5 und 10 $\mu$m) aus: 97% Au, 0,6% Ag, 0,4 % Pt und 2% Cu, Fe, Cd.

Das Bindemittel wird bei 1024°C 3 Minuten auf das Platinblech aufgebrannt. Danach wird eine Schicht eines handelsüblichen Dentalporzellans (Hersteller: Ceramco) aufgebracht und bei 969°C gebrannt. Der gefundene Verbund ist klinisch unzerbrechlich.

Entsprechende Versuche wurden mit anderen Dentalporzellanen durchgeführt, wobei vergleichbare Ergebnisse erhalten wurden.

Beispiel 2

Es wurde wie nach Beispiel 1 gearbeitet, wobei jedoch der Metallunterbau wie folgt geändert wurde:

1) 87,5% Au, 4,5% Pt, 6% Pd, Rest Spurenelemente;
2) 52% Au, 38% Pd, 4% Zn, Rest Spurenelemente;
3) 60% Ag, 40% Pd.

Die gefundenen Ergebnisse sind mit denen von Beispiel 1 vergleichbar.

Beispiel 3

Es wurde wie nach Beispiel 1 gearbeitet, wobei jedoch (A) 93% AgCl und (B) 7% der gleichen Edelmetallkomponente wie in Beispiel 1 verwendet wurden. Es wurden vergleichbare Ergebnisse erhalten.

Beispiel 4

Es wurde wie nach Beispiel 1 gearbeitet, wobei jedoch (A) 50% AgCl und (B) 50% der gleichen Edelmetallkomponente wie in Beispiel 1 verwendet wurden. Es wurden vergleichbare Ergebnisse erhalten.

Beispiel 5

Es wurde wie nach Beispiel 1 gearbeitet, wobei jedoch (A) 7% AgF und (B) 93% Au verwendet wurden. Der erhaltene Verbund ist klinisch unzerbrechlich.

Beispiel 6

Es wurde wie nach Beispiel 1 gearbeitet, wobei jedoch ausschließlich $PtCl_2$, d.h. keine Edelmetallkomponente, verwendet wurde. Es wurden vergleichbare Ergebnisse erhalten.

Beispiel 7

Es wurde wie nach Beispiel 1 gearbeitet, wobei jedoch (A) 10% $PtCl_2$ und (B) 90% Au verwendet wurden. Es wurden vergleichbare Ergebnisse erhalten.

Beispiel 8

Es wurde wie nach Beispiel 1 gearbeitet, wobei jedoch auschließlich $HAuCl_4$, d.h. keine Edelmetallkomponente als Bindemittel verwendet wurde. Es wurden vergleichbare Ergebnisse erhalten.

Beispiel 9

Es wurde wie nach Beispiel 1 gearbeitet, wobei jedoch (A) 10% $HAuCl_4$ und (B) 90% Au verwendet wurden. Es wurden vergleichbare Ergebnisse erhalten.

Beispiel 10

Es wurde wie nach Beispiel 1 gearbeitet, wobei jedoch (A) 5% $AgCl_2$ und 5% $PtCl_2$ und (B) 90% Au verwendet wurden. Es wurden vergleichbare Ergebnisse erhalten.

Beispiel 11

Es wurde wie nach Beispiel 1 gearbeitet, wobei jedoch (A) 10% $HauCl_4$ und 5% $PtCl_2$ und (B) 80% Au verwendet wurden. Der Rest (5%) bestand aus einem organischen Trägerstoff. Es wurden vergleichbare Ergebnisse erhalten.

Beispiel 12

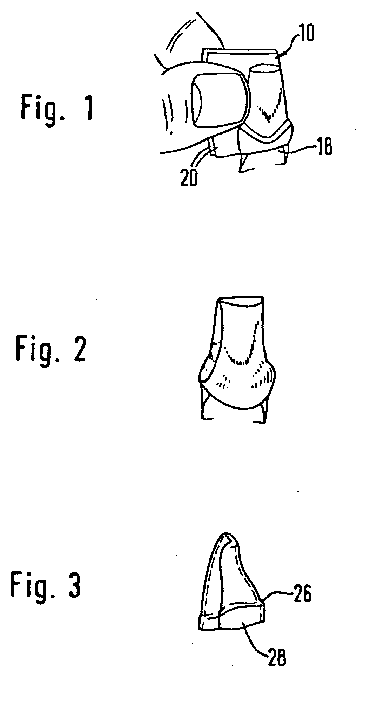

Ein Stumpfmodell 18 wird aus einem Abdruck des präparierten Zahnes hergestellt. Wie Fig. 1 zeigt, wickelt man eine Platinfolie 10 eng um den Stumpf 18 und formt so überlappende Enden 20, die wie in Fig. 2 erkennbar, gefaltet werden. Die Folie sollte über den Zahnfleischrand 26 reichen und eine Art Saum 28 bilden. Mittels einer Apparatur oder von Hand wird auf die Folie 10 ein Druck ausgeübt, um sie an den Stumpf 18 eng anzupressen. Anschließend zieht man sie vom Stumpf 18 und erhält eine Edelmetallunterlage, wie sie Fig. 3 zeigt.

Auf diesen Matallunterbau trägt man das Bindemittel gemäß Beispiel 8 auf und brennt bei 970°C 5 Minuten. Anschließend werden in bekannter Weise Dentalporzellanschichten aufgebracht und gebrannt.

Um die Festigkeit der fertigen Krone zu demonstrieren, befestigt man diese auf einen Metallstumpf und bringt einen Metallstift mit der incisalen Begrenzung der Krone in Berührung. Ein 100 g schwerer Metallzylinder wird aus einem Abstand von 20 cm über den Metallstift befestigt und fallengelassen. Es wird keine wesentliche Beschädigung der Krone festgestellt. Erhöht man den Abstand des Metallzylinders auf 40 cm, so bricht das Porzellan nur an einer begrenzten Fläche.

Zum Vergleich wird derselbe Test mit einer Krone gemacht, die ohne das erfindungsgemäße Bindemittel hergestellt wurde. Das 100 g-Gewicht verursacht aus einem Abstand von 20 cm das Herausbrechen von großen Dentalporzellanteilen.

**Patentansprüche**

1. Verwendung eines Mittels, enthaltend 1 bis 100 Gew.-% eines oder mehrerer Edelmetallhalogenide und 0 bis 99 Gew.-% einer feinteiligen Edelmetallkomponente, zum Verbinden von Dentalmetallteilen, insbesondere aus Edelmetallen oder Edelmetall-Legierungen, mit Dentalporzellan bzw. anderen Dentalmetallteilen bei Zahnrestaurationen.

2. Mittel zum Verbinden von Dentalmetallteilen, insbesondere aus Edelmetallen oder Edelmetall-Legierungen, mit Dentalporzellan bzw. anderen Dentalmetallteilen bei Zahnrestaurationen, dadurch gekennzeichnet, daß es 1 bis 99,9 Gew.-% eines oder mehrerer Edelmetallhalogenide und 0,1 bis 99 Gew.-% einer feinteiligen Edelmetallkomponente enthält.

3. Mittel nach Anspruch 2, dadurch gekennzeichnet, daß die Edelmetallhalogenide Chloride und/oder Fluoride darstellen.

4. Mittel nach Anspruch 2 oder 3, dadurch gekennzeichnet, daß die Edelmetallhalogenide Silberchlorid und/oder Goldfluorid darstellen.

5. Mittel nach einem der Ansprüche 2 bis 4, dadurch gekennzeichnet, daß die Edelmetallkomponente Gold und/oder Platin enthält oder daraus besteht.

6. Mittel nach einem der Ansprüche 2 bis 5, dadurch gekennzeichnet, daß die Edelmetallkomponente wenigstens 50 Gew.-% Gold enthält und der Rest zu 0 bis 45 Gew.-% aus einem oder mehreren der Metalle Silber, Platin, Palladium oder Rhodium und zu 0 bis 5 Gew.-% aus einem oder mehreren der Nichtedelmetalle Kupfer, Zink, Eisen, Zinn, Cadmium, Mangan, Germanium, Magnesium, Kobalt oder Nickel zusammengesetzt ist.

7. Mittel nach einem der Ansprüche 2 bis 6, dadurch gekennzeichnet, daß es zusätzlich Dispersions- und/oder Trägerstoffe enthält.

8. Mittel nach Anspruch 7, dadurch gekennzeichnet, daß der Trägerstoff durch Hitzeeinwirkung entfernbar ist.

9. Mittel nach einem der Ansprüche 2 bis 8, dadurch gekennzeichnet, daß die Teilchengröße der Edelmetallkomponente zwischen 1 und 60 $\mu$m liegt.

10. Verfahren zum Verbinden von Dentalmetallteilen, insbesondere aus Edelmetallen oder Edelmetall-Legierungen, mit Dentalporzellan bzw. mit anderen Dentalmetallteilen bei Zahnrestaurationen, wobei ein Bindemittel auf eine Metallunterlage aufgebracht und sintert bzw. gebrannt wird, dadurch gekennzeichnet, daß man ein Bindemittel verwendet, das 1 bis 100 Gew.-% eines oder mehrerer Edelmetallhalogenide und 0 bis 99 Gew.-% einer feinteiligen Edelmetallkomponente enthält.

11. Verfahren nach Anspruch 10, dadurch gekennzeichnet, daß man ein Bindemittel verwendet, das 1 bis 99,9 Gew.-% eines oder mehrerer Edelmetallhalogenide und 0,1 bis 99 Gew.-% einer feinteiligen Edelmetallkomponente enthält.

12. Verfahren nach Anspruch 10, dadurch gekennzeichnet, daß man eine Metallunterlage verwendet, die vorwiegend aus einem oder mehreren der Edelmetalle Gold, Silber, Platin und Palladium zusammengesetzt ist.

13. Verfahren nach einem der Ansprüche 10 bis 12, dadurch gekennzeichnet, daß man ein Bindemittel verwendet, dessen Edelmetallhalogenidkomponente Chloride und/oder Fluoride enthält.

14. Verfahren nach einem der Ansprüche 10 bis 13, dadurch gekennzeichnet, daß man ein Bindemittel verwendet, dessen Edelmetallhalogenidkomponente Silberchlorid, Goldchlorid und/oder Goldfluorid enthält.

15. Verfahren nach einem der Ansprüche 10 bis 14, dadurch gekennzeichnet, daß man ein Bindemittel verwendet, dessen Edelmetallkomponente Gold, Silber, Platin und/oder Palladium enthält.

16. Verfahren nach einem der Ansprüche 10 bis 15, dadurch gekennzeichnet, daß man ein Bindemittel verwendet, dessen Edelmetallkomponente wenigstens 50 Gew.-% Gold enthält und der Rest zu 0 bis 45 Gew.-% aus einem oder mehreren der Metalle Silber, Platin, Palladium oder Rhodium und zu 0 bis 5 Gew.-% aus einem oder mehreren der Nichtedelmetalle Kupfer, Zink, Eisen, Zinn, Cadmium, Mangan, Germanium, Magnesium, Kobalt oder Nickel zusammengesetzt ist.

17. Verfahren nach einem der Ansprüche 10 bis 16, dadurch gekennzeichnet, daß man das Bindemittel zusammen mit einem Dispersionsmittel und/oder einem Trägerstoff auf das Dentalmetallteil und/oder auf das Dentalporzellan aufbringt.

18. Verfahren nach einem der Ansprüche 10 bis 17, dadurch gekennzeichnet, daß man das Bindemittel im Temperaturbereich von 870 bis 1080°C sintert.

19. Verfahren nach einem der Ansprüche 10 bis 18, dadurch gekennzeichnet, daß man das Bindemittel 1 bis 25 min. sintert.

20. Verfahren nach einem der Ansprüche 10 bis 19, dadurch gekennzeichnet, daß man ein Bindemittel verwendet, dessen Edelmetallkomponente eine Teilchengröße zwischen 1 und 60 $\mu$m besitzt.

21. Verfahren nach einem der Ansprüche 10 bis 20, dadurch gekennzeichnet, daß man als Dentalmetallteile Edelmetallfolien, vorzugsweise Platinfolien, verwendet.

22. Verfahren nach einem der Ansprüche 10 bis 21, dadurch gekennzeichnet, daß man mehrere gleiche oder verschiedene Schichten Bindemittel auf ein Dentalmetallteil aufsintert.

## Revendications

1. Utilisation d'un produit renfermant 1 à 100% en poids d'un ou plusieurs halogénures de métaux précieux et 0 à 99% en poids d'un composant de métaux précieux en fines particules, pour lier des éléments dentaires métalliques, notamment constitués par des métaux précieux ou des alliages de métaux précieux, à de la porcelaine dentaire ou à d'autres éléments dentaires métalliques, dans des prothèses dentaires.

2. Produit pour lier des éléments dentairs métalliques, comprenant notamment des métaux précieux ou des alliages de métaux précieux, à de la porcelaine dentaire ou à d'autres éléments dentaires métalliques dans des prothèses dentaires, caractérisé en ce qu'il renferme 1 à 99,9% en poids d'un ou plusieurs halogénures de métaux précieux et 0,1 à 99% en poids d'un composant de métaux précieux en fines particules.

3. Produit selon la revendication 2, caractérisé en ce que les halogénures de métaux précieux sont des chlorures et/ou des fluorures.

4. Produit selon la revendication 2 ou 3, caractérisé en ce que les halogénures de métaux précieux sont le chlorure d'argent et/ou le fluorure d'or.

5. Produit selon l'une des revendications 2 à 4, caractérisé en ce que le composant de métaux précieux renferme de l'or et/ou du platine ou est constitué par ceux-ci.

6. Produit selon l'une des revendications 2 à 5, caractérisé en ce que le composant de métaux précieux renferme au moins 50% en poids d'or et en ce que le reste se compose pour 0 à 45% en poids d'un ou plusieurs métaux parmi l'argent, le platine, le palladium et le rhodium, et pour 0 à 5% en poids d'un ou plusieurs métaux non précieux parmi le cuivre, le zinc, le fer, l'étain, le cadmium, le manganèse, le germanium, le magnésium, le cobalt et le nickel.

7. Produit selon l'une des revendications 2 à 6, caractérisé en ce qu'il renferme en plus des matières de dispersion et/ou de support.

8. Produit selon la revendication 7, caractérisé en ce que la matière de support peut être éliminée sous l'action de la chaleur.

9. Produit selon l'une des revendications 2 à 8, caractérisé en ce que la dimension granulométrique du constituant de métaux précieux est comprise entre 1 et 60 microns.

10. Procédé pour lier des éléments dentaires métalliques, notamment constitués par des métaux précieux ou des alliages de métaux précieux, à de la porcelaine dentaire ou d'autres éléments dentaires métalliques dans des prothèses dentaires, en appliquant un liant à un substrat métallique, et en l'agglomérant par frittage ou en le cuisant, caractérisé en ce qu'on utilise un liant renfermant 1 à 100% en poids d'un ou plusieurs halogénures de métaux précieux et 0 à 99% en poids d'un composant de métaux précieux en particules fines.

11. Procédé selon la revendication 10, caractérisé en ce qu'on utilise un liant renfermant 1 à 99,9% en poids d'un ou plusieurs halogénures métalliques et 0,1 à 99% en poids d'un composant de métaux précieux en particules fines.

12. Procédé selon la revendication 10, caractérisé en ce qu'on utilise un substrat métallique composé surtout d'un ou plusieurs métaux précieux parmi l'or, l'argent platine et le palladium.

13. Procédé selon l'une des revendications 10 à 12, caractérisé en ce qu'on utilise un liant dont le composant d'halogénures de métaux précieux comprend des chlorures et/ou fluorures.

14. Procédé selon l'une des revendications 10 à 13, caractérisé en ce qu'on utilise un liant dont le composant d'halogénures de métaux précieux renferme du chlorure d'argent, du chlorure d'or et/ou du fluorure d'or.

15. Procédé selon l'une des revendications 10 à 14, caractérisé en ce qu'on utilise un liant dont le composant de métaux précieux renferme de l'or, de l'argent, du platine et/ou du palladium.

16. Procédé selon l'une des revendications 10 à 15, caractérisé en ce qu'on utilise un liant dont le composant de métaux précieux renferme au moins 50% d'or et dont le reste se compose pour 0 à 45% en poids d'un ou plusieurs métaux parmi l'argent, le platine, le palladium, ou le rhodium et pour 0 à 5% en poids d'un ou plusieurs métaux non-précieux parmi le cuivre, le zinc, le fer, l'étain, le cadmium, le manganèse, le germanium, le magnésium, le cobalt ou le nickel.

17. Procédé selon l'une des revendications 10 à 16, caractérisé en ce qu'on applique le liant en même temps qu'un agent de dispersion et/ou une matière de support à l'élément dentaire métallique et/ou à la porcelaine dentaire.

18. Procédé selon l'une des revendications 10 à 17, caractérisé en ce qu'on agglomère par frittage le liant dans un intervalle de température de 870 à 1.080°C.

19. Procédé selon l'une des revendications 10 à 18, caractérisé en ce qu'on agglomère par frittage le liant 1 à 25 minutes.

20. Procédé selon l'une des revendications 10 à 19, caractérisé en ce qu'on utilise un liant dont le composant de métaux précieux présente une dimension granulométrique comprise entre 1 à 60 microns.

21. Procédé selon l'une des revendications 10 à 20, caractérisé en ce qu'on utilise comme éléments dentaires métalliques des pellicules de métaux précieux, de préférence des pellicules d'or.

22. Procédé selon l'une des revendications 10 à 21, caractérisé en ce qu'on agglomère par frittage plusieurs couches de liant identiques ou différentes sur un élément dentaire métallique.

**Claims**

1. Use of an agent containing 1 to 100% by weight of one or more noble metal halides and 0 to 99% by weight of a finely divided noble metal component, for bonding dental metallic parts, in particular of noble metals or noble metal alloys, to dental porcelain or to other dental metallic parts during tooth restoration.

2. Agent for the bonding of dental metallic parts, in particular of noble metals or noble metal alloys, to dental porcelain or to other dental metallic parts during tooth restoration; characterised in that it contains 1 to 99.9% by weight of one or more noble metal halides and 0.1 to 99% by weight of a finely divided noble metal component.

3. Agent according to claim 2, characterised

in that the noble metal halides are constituted by chlorides and/or fluorides.

4. Agent according to claim 2 or claim 3, characterised in that the noble metal halides are constituted by silver chloride and/or gold fluoride.

5. Agent according to any of claims 2 to 4, characterised in that the noble metal component contains gold and/or platinum or consists thereof.

6. Agent according to any of claims 2 to 5, characterised in that the noble metal component contains at least 50% by weight of gold, the remainder being from 0 to 45% by weight composed of one or more of the metals silver, platinum, palladium or rhodium and from 0 to 5% by weight of one or more of the non-noble metals copper, zinc, iron, tin, cadmium, manganese, germanium, magnesium, cobalt or nickel.

7. Agent according to any of claims 2 to 6, characterised in that additionally it contains a dispersant and/or carrier.

8. Agent according to claim 7, characterised in that the carrier can be removed by the action of heat.

9. Agent according to any of claims 2 to 8, characterised in that the particle size of the noble metal component lies between 1 and 60 μm.

10. Method for the bonding of dental metallic parts, in particular of noble metals or noble metal alloys, to dental porcelain or to other dental metallic parts during tooth restoration, wherein a bonding agent is applied on to a metallic support and is sintered or stoved thereon, characterised in that a bonding agent is used which contains 1 to 100% by weight of one or more noble metal halides and 0 to 99% by weight of a finely divided noble metal component.

11. Method according to claim 10, characterised in that a bonding agent is used which contains 1 to 99.9% by weight of one or more noble metal halides and 0.1 to 99% by weight of a finely divided noble metal component.

12. Method according to claim 10, characterised in that a metallic support is used which is predominantly composed of one or more of the noble metals gold, silver, platinum and palladium.

13. Method according to any of claims 10 to 12, characterised in that a bonding agent is used, the noble metal halide component of which contains chlorides and/or fluorides.

14. Method according to any of claims 10 to 13, characterised in that a bonding agent is used, the noble metal halide component of which contains silver chloride, gold chloride and/or gold fluoride.

15. Method according to any of claims 10 to 14, characterised in that a bonding agent is used, the noble metal component of which contains gold, silver, platinum and/or palladium.

16. Method according to any of claims 10 to 15, characterised in that a bonding agent is used, the noble metal component of which contains at least 50% by weight of gold, the remainder being from 0 to 45% by weight composed of one or more of the metals silver, platinum, palladium or rhodium and from 0 to 5% by weight of one or more of the non-noble metals copper, zinc, iron, tin, cadmium, manganese, germanium, magnesium, cobalt or nickel.

17. Method according to any of claims 10 to 16, characterised in that the bonding agent is applied, together with a dispersant and/or carrier, on to the dental metallic part and/or on to the dental porcelain.

18. Method according to any of claims 10 to 17, characterised in that the bonding agent is sintered in the temperature range of 870 to 1080°C.

19. Method according to any of claims 10 to 18, characterised in that the bonding agent is sintered for 1 to 25 minutes.

20. Method according to any of claims 10 to 19, characterised in that a bonding agent is used, the noble metal component of which has a particle size of between 1 and 60 μm.

21. Method according to any of claims 10 to 20, characterised in that noble metal foils, in particular platinum foils, are used as dental metallic parts.

22. Method according to any of claims 10 to 21, characterised in that several like or different layers of bonding agent are sintered on to a dental metallic part.

Fig. 1

Fig. 2

Fig. 3